# EUROPEAN PATENT SPECIFICATION

(11) **EP 1 693 060 B1**
(45) Date of publication and mention of the grant of the patent: **02.05.2012**
(21) Application number: 04799962.8
(22) Date of filing: 30.11.2004
(51) Int. Cl.: A61K 31/352, C07D 311/86, A61P 25/00, A61P 27/02, A61P 27/06, A61P 9/10, C12P 17/06, A61K 31/353

(54) **THERAPEUTIC OR PREVENTIVE AGENTS FOR ISCHEMIC NEUROPATHY**
MITTEL ZUR BEHANDLUNG ODER PRÄVENTION VON ISCHÄMISCHER NEUROPATHIE
AGENTS THERAPEUTIQUES OU PREVENTIFS DESTINES A LA NEUROPATHIE ISCHEMIQUE

(30) Priority: 05.12.2003 JP 2003406804
(43) Date of publication of application: 23.08.2006
(73) Proprietor: Dainippon Sumitomo Pharma Co., Ltd., Osaka 541-8524 (JP)
(72) Inventor: IKEDA, Kazuhito, c/o Dainippon Sumitomo Pharma, Osaka 564-0053 (JP); KIMURA, Toru, c/oDainippon Sumitomo Pharma, Osaka-shi, Osaka 554-0022 (JP)
(74) Representative: Duckworth, Timothy John
(86) International application number: PCT/JP2004/018114
(87) International publication number: WO 2005/053678

(56) References cited:
- EP-A- 1 306 093
- WO-A-03/062243
- WO-A-03/062440
- WO-A1-02/09756
- JP-A- 6 506 202
- JP-A- 8 165 237
- US-A1- 2002 040 052
- FENG GUI-LONG ET AL: "Protection of methylflavonolamine against acute cerebral ischemia reperfusion injury in rats" ACTA PHARMACOLOGICA SINICA, vol. 16, no. 4, 1995, pages 304-307, XP002483823 ISSN: 0253-9756
- PLOTNIKOV M B ET AL: "Cerebroprotective effects of diquertin and ascorbic acid." BULLETIN OF EXPERIMENTAL BIOLOGY AND MEDICINE NOV 2000, vol. 130, no. 11, November 2000 (2000-11), pages 1080-1083, XP002483824 ISSN: 0007-4888
- WANG LEIYI ET AL: "Protective effect of Puerarin on acute cerebral ischemia in rats" ZHONGGUO ZHONGYAO ZAZHI - CHINA JOURNAL OF CHINESE MATERIA MEDICA, ZHOGGUO YAOXUEHUI, BEIJING, CN, vol. 22, no. 12, 1 December 1997 (1997-12-01), pages 752-754,765, XP009101476 ISSN: 1001-5302
- SHIRVAN ANAT ET AL: "Anti-semaphorin 3A antibodies rescue retinal ganglion cells from cell death following optic nerve axotomy." THE JOURNAL OF BIOLOGICAL CHEMISTRY 20 DEC 2002, vol. 277, no. 51, 20 December 2002 (2002-12-20), pages 49799-49807, XP002483825 ISSN: 0021-9258
- SOLOMON ARIEH S ET AL: "Up-regulation of semaphorin expression in retina of glaucomatous rabbits" GRAEFE'S ARCHIVE FOR CLINICAL AND EXPERIMENTAL OPHTHALMOLOGY, SPRINGER VERLAG, XX, vol. 241, no. 8, 1 August 2003 (2003-08-01), pages 673-681, XP002443105 ISSN: 0721-832X
- KUMAGAI KAZUO ET AL: "Xanthofulvin, a novel semaphorin inhibitor produced by a strain of Penicillium" JOURNAL OF ANTIBIOTICS, JAPAN ANTIBIOTICS RESEARCH ASSOCIATION, TOKYO, JP, vol. 56, no. 7, 1 July 2003 (2003-07-01), pages 610-616, XP009101444 ISSN: 0021-8820
- GAMMON G ET AL: 'A fungal metabolite which inhibits the interaction of CD4 with major histocompatibility complex-encoded class II molecules' EUROPEAN JOURNAL OF IMMUNOLOGY vol. 24, no. 4, 1994, pages 991 - 998, XP009015534
- KIKUCHI K ET AL: 'In vitro and in vivo characterization of a novel semaphorin 3A inhibitor, SM-216289 or Xanthofulvin' JOURNAL OF BIOLOGICAL CHEMISTRY vol. 278, no. 44, 2003, pages 42985 - 42991, XP002985089
- NAKAYAMA T ET AL: 'Role of Cdk5 and tau phosphorylation in heterotrimeric G protein-mediated retinal growth cone collapse' JOURNAL OF NEUROBIOLOGY vol. 41, no. 3, 1999, pages 326 - 339, XP002985099

## Description

### TECHNICAL FIELD

The present invention relates to therapeutic or preventive agents for ischemic nerve injury containing a compound obtained by culturing Penicillium sp. SPF-3059 strain or a pharmaceutically acceptable salt thereof as an active ingredient.

### BACKGROUND ART

It has been reported that ischemia is involved in some cytopathic diseases of the central nervous system and the peripheral nervous system. Ischemic disorder is also considered to be involved in retinal nerve diseases such as glaucoma, central retinal artery occlusion, branch retinal artery occlusion, central retinal vein occlusion, branch retinal vein occlusion, ischemic optic neuropathy, diabetic retinopathy, macular degeneration and retinopathy of prematurity.

### (1) Glaucoma

Glaucoma is a retinal disease having an incidence of 3.5% among people not younger than 40 years old, and 1.92 million in Japan, 105 million patients in the world are estimated to suffer from glaucoma. The main symptom is increased intra ocular pressure, and retinal atrophy and retraction of optic papilla are caused. Glaucoma results in a poor prognosis and may lead to blindness in the worst case, and it is the fourth leading cause of blindness in Japan and the patients as many as 120,000 a year are reported in the U.S.A.

Since increased intra ocular pressure is a main symptom of glaucoma, a large number of drugs aiming at decreasing intra ocular pressure have been developed. They can be classified into five types: parasympathomimetic agents, sympathomimetic agents, β sympatholytic agents, carbonic anhydrase inhibitors and hyperosmotic agents according to the difference in the site of action, but all these agents exhibit drug efficacy by decreasing an intra ocular pressure. Prognosis is still poor and the optic nerve is damaged in some cases, even when an intra ocular pressure is lowered. In addition, a lot of cases of glaucoma without rise in intra ocular pressure, namely low tension glaucoma have been reported, and the number of patients with low tension glaucoma is estimated to be one million, while that of glaucoma associated with rise in intra ocular pressure is estimated to be 2 to 3 millions in the U.S.A. Accordingly, drugs not aiming at decreasing intra ocular pressure but directly bringing about a protective effect to optic nerve cells are expected.

### (2) Central retinal artery occlusion

Central retinal artery occlusion is a disease developed by lodging of a thrombus at a passage point in cribrosa lamina of the central retinal artery. As a symptom, a sudden and unilateral fall in visual acuity occurs and atrophy of the optic nerve is caused. Unlike chronic ischemia, angiogenesis is not observed later. Because the central retinal artery is the end artery, acute ischemia lasting for 30 to 40 minutes causes an irreversible change and leads to necrosis of the retina. Therefore, eyesight prognosis is poor in the case of complete occlusion.

Treatment is to perform eye massage immediately after the symptom is noticed and to try for blood flow resumption. As for drug therapy, urokinase + dextran are used for thrombolysis and prostaglandin E₁ (alprostadil) for vasodilation and/or prevention of thrombogenesis.

### (3) Branch retinal artery occlusion

Branch retinal artery occlusion is a disease in which a thrombus lodges at an intraocular branching part and a disorder develops only in the area controlled thereby. Treatment is performed similarly as for central retinal artery occlusion.

### (4) Central retinal vein occlusion / Branch retinal vein occlusion

Both of central retinal vein occlusion, which is classified into hemorrhagic retinopathy and venous stasis retinopathy according to the presence or absence of hemorrhage, and branch retinal vein occlusion are caused by a thrombus which occurred in the cribrosa lamina.

Hemorrhagic retinopathy occurs common by among elderly people, and is caused by arterial sclerosis in more than half. As a symptom, flame-like hemorrhage spreads along running of the nerve fibers from to optic papilla in the retina cortex, and remarkable decrease of eyesight is caused.

As treatment, urokinase + dextran are used similarly as for central retinal artery occlusion and carbazochrome sodium sulfonate or adrenochrome guanylhydrazone mesilate is used for blood vessel reinforcement. Laser photocoagulation is also performed for preventing macula retinae edema or angiogenesis.

As for venous stasis retinopathy, one caused by inflammation and one caused by arterial sclerosis are known, and the former is common among young people while the latter is common among elderly people. As a symptom, strong dilation and meandering of the veins and further, flare of the optic papilla occur, but a decrease in visual acuity is slight as compared with the hemorrhagic type.

As treatment, dry therapy the same as that for the hemorrhagic type is performed, but laser photocoagulation is not adopted.

### (5) Ischemic optic neuropathy

Ischemic optic neuropathy is a disease in which necrosis of the optic nerves occur by occlusion of nutrient blood vessels and dysfunction in visual performance appears, and it is also called as anterior ischemic optic neuropathy.

It is divided, according to causes, into temporal arteritis and idiopathic ischemic optic neuropathy developed by systemic diseases such as arterial sclerosis, hypertension and diabetes mellitus.

Only drug therapy is adopted. A steroid (prednisolone) is used to improve blood flow around the cribrosa lamina, an intra ocular pressure depressant (carbonic anhydrase inhibitor, acetazolamide) is used to reduce edema, and vitamin B₁ and B₁₂ are used to stimulate the nerve.

### (6) Diabetic retinopathy

Diabetic retinopathy develops following hyperglycemia persisting for several years or more, which causes occlusion of minute vessels from degeneration of endothelial cells of the minute vessels, necrosis, thrombogenesis, and enhancement blood clotting ability and leads to an ischemic state. This disease occurs in 39% of patients with insulin-dependent disease and 47% of patients with insulin-independent disease, and the number of patients in Japan is estimated to be about 600 thousand.

As treatment, a glucose level is systemically controlled at the simple stage, a blood vessel enhancing agent and a streptokinase streptodornase (Varidase) are adopted in the case associated with hemorrhage, and panretinal photocoagulation is performed at the preproliferative stage. Furthermore, an operation is performed on the vitreous body when traction retinal detachment occurs in the proliferative stage. No drugs aiming at protection of the retinal nerve are available, and tocopherol calcium succinate (vitamin E), and tocopherol acetate (Juvela) are adopted only secondarily. Therefore, development of a new drug alleviating ischemia disorder is expected.

### (7) Macular degeneration

Macular area is the retinal part at which the optical axis passes and is an important area carrying the center of viewing field and deciding visual acuity. Macular degeneration is a generic designation of symptoms which cause abnormality on this part, and five diseases are mainly known. That is, it is classified into central serious chorioret, central exudative chorioretinopathy, senile disciform macular degeneration, senile atrophic macular degeneration and idiopathic vitreoretinal interface maculopathy.

Central serious chorioret and central exudative chorioretinopathy are diseases in which serous fluid or hemorrhage (exudate) passes through degenerated retinal pigmentary epithelial cells and stays below the retina.

Senile disciform macular degeneration is a disease which causes exudative alteration and hemorrhage in the macular area and angiogenesis from choroid while senile atrophic macular degeneration causes no observable exudative alteration and is characterized by atrophy of retinal pigmentary epithelial cells.

In idiopathic vitreoretinal interface maculopathy, a transparent or opaque preretinal membrane is formed in the macular area, and blood vessels running to the macular area become serpiginous.

Since each of the diseases is caused by vascular disorder, except for senile atrophic macular degeneration, they may be attributable to ischemic injury.

As treatment, photocoagulation is basically performed when angiogenesis occurs, and as drug therapy, a highly penetrating agent (isosorbyl and glycerine) is used for absorption of the serous fluid below the retina, kallidinogenase is used to improve circulation in the retina and choroid, streptokinase streptodornase (Varidase) is used for vasodilation, and further, vitamins and steroids are adopted, but there is no drug acting on the retinal nerve cells, and development of a new drug is expected.

### (8) Retinopathy of prematurity

Retinopathy of prematurity is caused by high concentration oxygenation to a prematurely born child, which causes obliterative alteration in the peripheral region of the immature retinal vessels and leads to anoxia after the oxygenation is terminated. There is no treatment but symptomatic treatment such as cryocoagulation, and there is no drug for radical remedy, and therefore, development of a new drug which reduces disorder at the time of ischemia is expected.

In addition, injury by ischemia is also involved in cranial nerve diseases or cerebrovascular disorder such as cerebral embolism, transient cerebral ischemia, subclavian steal syndrome, Wallenberg syndrome (lateral medullary syndrome), cerebral thrombosis, lacunar infarct, reversible ischemic neurological deficit, cerebral infarction, moyamoya disease (occlusion of the circle of Willis), hypoxic encephalopathy, sinus venosus thrombosis and postoperative spinal cord ischemia, and development of a new drug which reduces injury at the time of ischemia in these diseases is expected.

In the meantime, semaphorin is an endogenous protein identified as a factor which collapses the nerve growth cone and suppresses axon elongation, and so far, about 20 molecular species have been reported and the most studied is the gene group of a subfamily called the class III type. The proteins encoded by these genes are known to possess intensive neurite outgrowth suppressing activity and growth cone collapse activity in vitro. Among them, semaphorin 3A (Sema3A) is the most studied (See Cell 75, p217, 1993, Cell 75, p1389, 1993) and is known to induce growth cone collapse of the cultured nerve cells at as low as 10 pM concentration in a short period of time. It is also known that anti-semaphorin 3A antibody suppresses cell death (apoptosis) of retinal gangliocyte in a rat optic nerve axotomy model, (See The Journal of Biological Chemistry, 277, p49799 (2002)). The above model is an injury disorder model in which only the axons (a nerve fiber) are cut off, and nerve vasculature or blood supply to the optic nerve is not damaged. Furthermore, it is known that expression of semaphorin is elevated in the retina of the congenital glaucoma model using a rabbit (See Graefe's Archive for Clinical and Experimental Ophthalmology, 241, p673 (2003)). In the meantime, it is known that mRNA of semaphorin 3A is expressed in the middle cerebral artery occlusion rat model (See Brain Research, 914, p1 (2001)).

It is known that a series of xanthone compounds has semaphorin inhibitory activity and has a nerve regeneration acceleration effect (See WO02/09756, WO03/062243 and WO03/062440). It has not been known, however, that these compounds having semaphorin inhibitory activity suppress cell death (apoptosis) at the time of ischemia, for example, cell death of the retinal nerves and nerve cell death in the brain, and show an excellent effect as a therapeutic or preventive agent effective for ischemic injury.

### DISCLOSURE OF THE INVENTION

An object of the present invention is to provide a therapeutic or preventive agent for nerve injury associated with ischemic injury.

The present inventors have found that a compound having semaphorin inhibitory activity suppresses nerve cell death involved with ischemic injury and is useful as a therapeutic or preventive agent for ischemic nerve injury.

That is, intra ocular pressure was increased by loading a pressure of about 150 mmHg on the anterior chamber of a rat with a sphygmomanometry device to prepare a model animal in which an ischemic injury was caused. When a compound having a semaphorin inhibitory activity obtained by culturing Penicillium sp. SPF-3059 strain was administered to the model animal, an excellent therapeutic effect was shown. Accordingly, the compound having a semaphorin inhibitory activity has been found to be effective as a therapeutic or preventive agent for ischemic injury, preferably as a therapeutic or preventive agent for ischemic injury in the retina.

That is, the present invention relates to
[1] Use of a compound having a semaphorin inhibitory activity which is obtained by culturing Penicillium sp. SPF-3059 strain and represented by formula [22]: 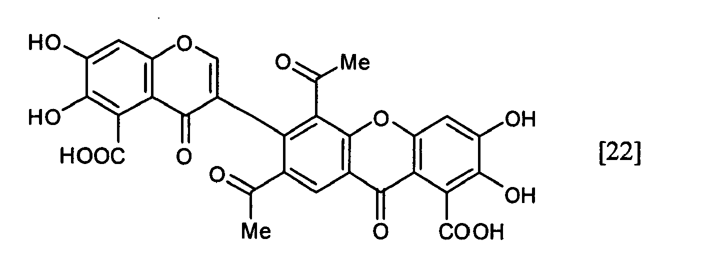 or a pharmaceutically acceptable salt thereof; in the manufacture of a medicament for use as an active ingredient in a therapeutic or preventive agent for suppressing nerve cell death in an ischemic nerve injury by ischemic disorder.
[2] The use of a compound or a pharmaceutically acceptable salt thereof in the manufacture of a medicament according to [1] wherein the ischemic disorder is cerebral embolism, transient cerebral ischemia, subclavian steal syndrome, Wallenberg syndrome (lateral medullary syndrome), cerebral thrombosis, lacunar infarct, reversible ischemic neurological deficit, cerebral infarction, moyamoya disease (occlusion of the circle of Willis), hypoxic encephalopathy, sinus venosus thrombosis or postoperative spinal cord ischemia.
[3] The use of a compound or a pharmaceutically acceptable salt thereof in the manufacture of a medicament according to [1] wherein the ischemic disorder is glaucoma, central retinal artery occlusion, branch retinal artery occlusion, central retinal vein occlusion, branch retinal vein occlusion, ischemic optic neuropathy, diabetic retinopathy, macular degeneration or retinopathy of prematurity.
[4] The use of a compound or a pharmaceutically acceptable salt thereof in the manufacture of a medicament according to [1] wherein the ischemic disorder is diabetic retinopathy.
[5] A compound having a semaphorin inhibitory activity which is obtained by culturing Penicillium sp. SPF-3059 strain and represented by formula [22] : 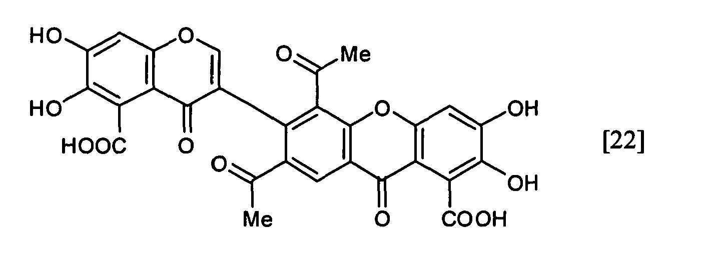 or a pharmaceutically acceptable salt thereof; for use as an active ingredient in a therapeutic or preventive agent for suppressing nerve cell death in an ischemic nerve injury by ischemic disorder.
[6] The compound or a pharmaceutically acceptable salt thereof for use as an active ingredient in a therapeutic or preventive agent according to [5], wherein the ischemic disorder is cerebral embolism, transient cerebral ischemia, subclavian steal syndrome, Wallenberg syndrome (lateral medullary syndrome), cerebral thrombosis, lacunar infarct, reversible ischemic neurological deficit, cerebral infarction, moyamoya disease (occlusion of the circle of Willis), hypoxic encephalopathy, sinus venosus thrombosis or postoperative spinal cord ischemia.
[7] The compound or a pharmaceutically acceptable salt thereof for use as an active ingredient in a therapeutic or preventive agent according to [5], wherein the ischemic disorder is glaucoma, central retinal artery occlusion, branch retinal artery occlusion, central retinal vein occlusion, branch retinal vein occlusion, ischemic optic neuropathy, diabetic retinopathy, macular degeneration or retinopathy of prematurity.
[8] The compound or a pharmaceutically acceptable salt thereof for use as an active ingredient in a therapeutic or preventive agent according to [5], wherein the ischemic disorder is diabetic retinopathy.

### BRIEF DESCRIPTION OF THE DRAWINGS

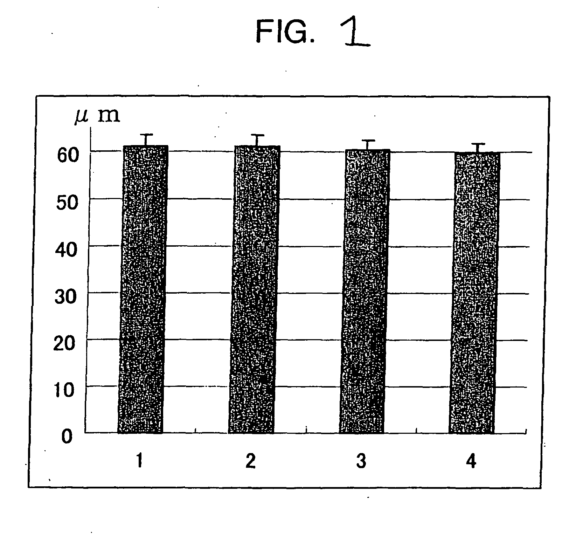

Fig. 1 shows the result of measuring the thickness of outer nuclear layer (ONL) [Student's t-test, compared to (IOP increase load +PBS administered) Group,*:<0.05 ]. The meanings of the numbers of the abscissa axis are as follows. That is, 1: normal intra ocular pressure; 2: intra ocular pressure (IOP) increase load +PBS administration; 3: IOP increase load + SPF-3059-5 pre-administration treatment; 4: IOP increase load + SPF-3059-5 post-administration treatment. It can be understood from Fig. 1 that the increased intra ocular pressure load or the administration of SPF-3059-5 does not have an influence on the outer nuclear layer;

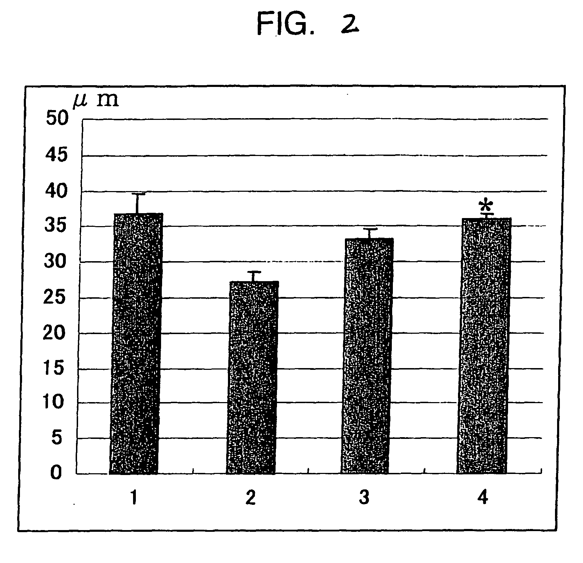

Fig. 2 shows the result of measuring the thickness of inner nuclear layer (INL) [Student's t-test, compared to (IOP increase load +PBS administered) Group,*:<0.05 ]. The meanings of the numbers 1 to 4 of the abscissa axis are the same as in Fig.1. It can be understood from Fig.2 that the thickness of INL which is made thinner by increased intra ocular pressure load is suppressed by administration of a drug;

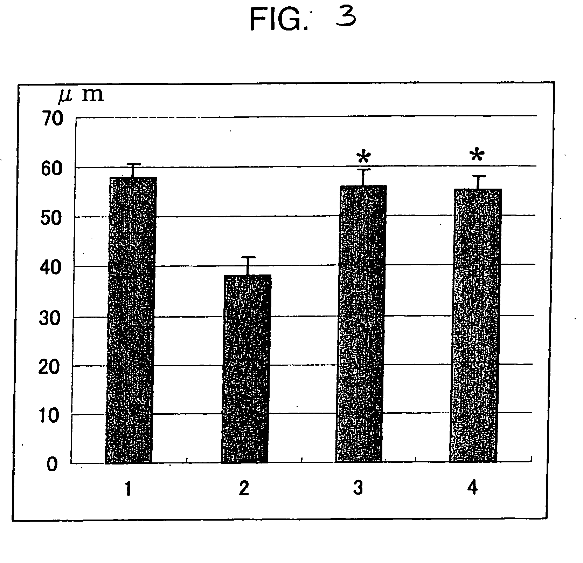

Fig.3 shows the result of measuring the thickness of inner plexus layer (IPL) [Student's t-test, compared to (IOP increase load +PBS administered) Group*:<0.05 ]. The meanings of the numbers 1 to 4 of the abscissa axis are the same as in Fig.5. It can be understood from Fig.3 that.the thickness of IPL which is made thinner by increased intra ocular pressure load is suppressed by administration of a drug; and

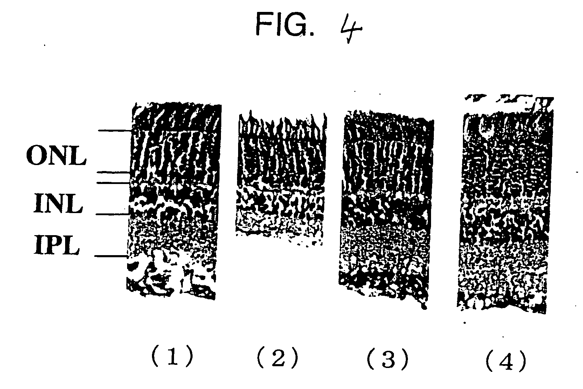

Fig.4 is a photograph of the retinal section dyed with 0.5% cresylviolet;
(1) Normal intra ocular pressure;
(2) Intra ocular pressure (IOP) increase load +PBS administration;
(3) IOP increase load + SPF-3059-5 pre-administration treatment;
(4) IOP increase load + SPF-3059-5 post-administration treatment.

### BEST MODE FOR CARRYING OUT THE INVENTION

The compound of, formula [22] is also referred to herein as SPF-3059-5.

The compound SPF-3059-5 can be obtained from a culture of Penicillium sp. SPF-3059 strain using semaphorin inhibitory activity as an indicator. In addition, it can be identified using semaphorin inhibitory activity as an indicator from the compounds produced by a known conversion method or a known synthesis method from the thus obtained compounds having semaphorin inhibitory activity.

As for the compound SPF-3059-5 salts which are pharmaceutically acceptable are included in the scope of the present invention. Here, the salts include an inorganic base salt such as sodium salt, potassium salt, calcium sodium, magnesium salt, aluminum salt and ammonium salt, an organic base salt such as triethylammonium salt, triethanol ammonium salt, pyridinium salt and diisopropyl ammonium salt, a salt of a basic amino acid such as arginine and lysin.

The compound SPF-3059-5 mentioned above can be effectively obtained by culturing a fungus SPF-3059 strain belonging to the genus Penicillium each separated from the soil in Osaka [This strain was deposited on Jul. 13, 2001 to the International Patent Organism Depositary, the National Institute of Advanced Industrial Science and Technology, an independent administrative institution of Ministry of Economy, Trade and Industry (Central 6, 1-1 Higashi 1-chome, Tsukuba, Ibaraki 305-8566, Japan) under the accession number FERM BP-7663 as the International Deposition Number under the Budapest Treaty on the international recognition of the deposit of microorganisms for the purpose of patent procedure], and can be obtained according to methods described in WO02/0975 or WO03/062243.

The compound having semaphorin inhibitory activity showed inhibitory effect on nerve cell death by ischemic disorder, and therefore, can be used as a therapeutic agent or preventive agent for ischemic neurological diseases. The ischemic neurological diseases as used herein include retinal neuropathy by ischemia or ischemic cerebrovascular diseases. Examples of the retinal neuropathy as used herein include glaucoma, central retinal artery occlusion, branch retinal artery occlusion, central retinal vein occlusion, branch retinal vein occlusion, ischemic optic neuropathy, diabetic retinopathy, macular degeneration, retinopathy of prematurity, and, above all, diabetic retinopathy is preferable. Examples of the ischemic cerebrovascular diseases include cerebral embolism, transient cerebral ischemia, subclavian steal syndrome, Wallenberg syndrome (lateral medullary syndrome), cerebral thrombosis, lacunar infarct, reversible ischemic neurological deficit, cerebral infarction, moyamoya disease (occlusion of the circle of Willis), hypoxic encephalopathy, sinus venosus thrombosis or postoperative spinal cord ischemia. The compound of the present invention has retinal nerve protective action and is particularly effective for treatment or prevention of retinal neuropathy by ischemia.

To the therapeutic or preventive agent for ischemic nerve injury of the present invention, various dispensing formulation ingredients may be added such as pharmaceutically acceptable ordinary carriers, binders, stabilizers, excipients, diluents, pH buffers, disintegrating agents, solubilizers, dissolving coadjuvants, isotonic agents or the like. Besides, the therapeutic or preventive agent can be administered either orally or parenterally. In other words, they can be administered in usual administration means, for example, they can be orally administered in agent forms such as tablet, pill, powder, granule, capsule, syrup, emulsion, suspension liquid.

They can be parenterally administered agent forms such as intravenous injection (drops), intramuscular injection, subcutaneous injection, eye drop, eye ointment.

Solid preparations such as tablets can be prepared by mixing an active ingredient with a pharmacologically acceptable ordinary carrier or excipient such as lactose, sucrose, corn starch, a binder such as hydroxypropylmethyl cellulose, polyvinylpyrrolidone, hydroxypropylcellulose, a disintegrating agent such as carboxymethylcellulose sodium or sodium carboxymethyl starch, a lubricant such as, stearic acid or magnesium stearate or a preservative, etc.

For parenteral administration, the active ingredient is dissolved or suspended in a physiologically acceptable carrier such as water, physiological saline solution, oil, aqueous glucose solution, which may contain an emulsifier, a stabilizer, a salt for osmoregulation or a buffer as an adjuvant as required. As an additive for eye drop, an isotonizing agent such as glycerine or sodium chloride, a buffer such as phosphoric acid or citric acid, a pH regulator such as hydrochloric acid or sodium hydroxide, a thickener such as hydroxypropylmethyl cellulose or polyvinyl alcohol, a preservative such as benzethonium chloride or a solubilizer may be contained as required. Examples of additives for eye ointment include petrolatum, polyethylene glycol, purified lanolin, and liquid paraffin.

Although dosage and frequency of administration vary depending on the method of administration and the age, weight, medical conditions or the like of a patient, local administration to the site of disease is preferable. It is preferable to administer once, twice or more per day. When it is administered twice or more, it is desirably administered repeatedly every day or at appropriate intervals.

Dosage may be several hundred µg to 2 g, preferably 5 to 100 mg, more preferably less than several tens mg per dose per adult patient as the active ingredient, and it can be administered once a day or divided into several times a day. When parenterally administered, dosage may be 0.1 to 100 mg, more preferably 0.3 to 50 mg per day per an adult patient and it can be administered once a day or divided into several times a day. In order to reduce administration frequency, a sustained release preparation may be used. When used as an eye drop, 0.0.1 to 10 w/v%, preferably 0.05 to 5 w/v% per adult patient as the active ingredient can be used and it is desirable to administer one to several drops per dose, 1 to 6 times a day, depending on the condition. When used as an eye ointment, 0.01 to 10 w/w%, preferably 0.1 to 5 w/w% as the active ingredient can be used and it is preferably to administer 1 to 6 times a day, depending on the condition.

In addition, the therapeutic or preventive agent for ischemic nerve injury of the present invention can be also used as veterinary drug.

### Examples

The present invention is now explained in details by way of examples but the technical scope of the invention is not to be limited to these examples.

### Example 1

### Pharmacological effect on an increased intra ocular pressure model

### Test method

### (1) Experimental animal

20 SD rats (7-week old) were used and divided into the following groups.
Sham operation: 4 rats
IOP (Intra Ocular pressure) treatment +PBS (5 µl was administered to the vitreous body): 4 rats
IOP (Intra Ocular pressure) treatment + test compound (5 µl was pre-administered to the vitreous body): 4 rats
IOP (Intra Ocular pressure) treatment + test compound (5 µl was post-administered to the vitreous body): 4 rats

### (2) Increase in intra ocular pressure

Increased intra ocular pressure model animals were prepared and a drug (test compound) was administered thereto .

### (3) Preparation of a drug and administration method

SPF-3059-5 as a test substance was diluted with PBS to 0.1 mg/ml immediately before use. 5 µl of the diluted solution was administered to the vitreous body of the left eye of the rats with a 30G double needle.

### (4) Significance test

Intra ocular pressure was increased by the method of above (2) and significance tests were conducted by Student's t-test and Welch & F-test on the retinal protection effect of SPF-3059-5 administration in rats to which PBS was administered to the vitreous body.

### Pharmacological evaluation

Each of the thickness of the outer nuclear layer, inner nuclear layer and inner plexus layer was measured using the retina of the rats in each treatment group and the cytoprotective effect was evaluated. The measured values were shown in Table 1 and Fig.1 (ONL: outer nuclear layer), Table 2 and Fig. 2 (INL: inner nuclear layer) and Table 3 and Fig. 3 (IPL: inner plexus layer). In addition, a retinal section dyed with 0.5% cresylviolet was shown in Fig. 4.

**Table 1**

| | Average (µm) | SD | SE |
|---|---|---|---|
| Sham ope | 61.76 | 3.40 | 1.70 |
| IOP increase load + PBS administration | 61.03 | 4.41 | 2.21 |
| IOP increase load + test substance pre-administration | 60.29 | 3.80 | 1.90 |
| IOP increase load + test substance post-administration | 61.76 | 4.16 | 2.08 |

**Table 2**

| | Average (µm) | SD | SE |
|---|---|---|---|
| Sham ope | 36.76 | 5.63 | 2.82 |
| IOP increase load + PBS administration | 27.21 | 2.82 | 1.41 |
| IOP increase load + test substance pre-administration | 33.09 | 2.82 | 1.41 |
| IOP increase load + test substance post-administration | 36.76 | 1.70 | 0.85 |

**Table 3**

| | Average (µm) | SD | SE |
|---|---|---|---|
| Sham ope | 58.09 | 5.02 | 2.51 |
| IOP increase load + PBS administration | 38.24 | 7.20 | 3.60 |
| IOP increase load + test substance pre-administration | 55.88 | 7.20 | 3.60 |
| IOP increase load + test substance post-administration | 55.15 | 6.52 | 3.26 |

It is known that outer granular cells receive blood supply from the choroidal layer and understood that the cells do not suffer from ischemic damage of ophthalmic artery due to increased intra ocular pressure, and according to the results of Table 1 and Fig. 1, it was shown that the thickness of the outer nuclear layer was not affected by increased intra ocular pressure in the present examples. Based upon this, it can be confirmed that the section made in this experiment is adequately prepared.

It is known that inner granular cells receive blood supply from ophthalmic artery and understood that the cells suffer from ischemic damage of ophthalmic artery due to increased intra ocular pressure, which causes delayed cell death. According to the results of Table 2 and Fig. 2, it was observed that increased intra ocular pressure caused cell death of the inner granular cells and the cell layer in which the cells were present became thinner in the present examples. Furthermore, although no statistically significant difference was recognized, it was observed that decrease of the thickness of the cell layer tends to be suppressed by pre-administration of SPF-3059-5, and cell death suppression effect on inner granular cell death was considered. In addition, cell death suppression effect by post-administration of SPF-3059-5 on inner granular cell death was shown statistically significantly.

Furthermore, the inner plexus layer is a layer where synapse formation between inner granular cells and gangliocytes is made, and the inner plexus layer is a layer where there is only axons extending from cell bodies. Since the inner granular cells suffer from ischemic injury of ophthalmic artery by increased intra ocular pressure, it is known that the inner plexus, the axial filaments thereof, does regress, and the inner plexus layer become thinner. According to the results of Table 3 and Fig. 3, this phenomenon was reproduced in this example, and decrease of the thickness of the inner plexus layer by increased intra ocular pressure was observed. Furthermore, it was shown that decrease of the thickness of the inner plexus layer was suppressed significantly by pre-administration and post-administration of SPF-3059-5, and considered that they protected a neurological function in the retina.

As a result of the above, it was shown that SPF-3059-5 suppressed cell death of inner granular cells due to increased intra ocular pressure. In addition, the inner plexus layer is a place where inner granular cells and gangliocytes form synapse and contains axons of gangliocyte and protection effect on gangliocyte was therefore also thought of.

In addition, it was shown that pre-administration treatment with SPF-3059-5 showed suppressing tendency for the inner granular cell layer and had suppressing effect on the decrease of the thickness of the inner plexus layer statistically significantly. Furthermore, it was found that post-administration treatment with SPF-3059-1 also had suppressing effect on the decrease of the thickness of inner granular cell layer and the inner plexus layer statistically significantly.

From the result described above, it has been found in ophthalmopathy that SPF-3059-5 is effective for diabetic retinopathy in which ischemic injury of inner granular cells is suspected and glaucoma in which injury of gangliocyte is reported, etc.

### Example 2

### Production of compound

The compound of the present invention is a known compound and is disclosed by WO02/09756 or WO03/062243 and can be produced from SPF-3059 strain which is a fungus belonging to the genus Penicillium. Production process and physico-chemical properties are described in the above international publication pamphlets.

### (Compound SPF-3059-5)

### Appearance: Cream-colored powder

High resolution fast atom bombardment mass spectrum (HRFAB-MS) m/z (M + H)⁺:
Observed value: 577.0615
Calculated value: 577.0619
Molecular formula: C₂₈H₁₆O₁₄
Ultraviolet visible absorption spectrum λ max (in methanol) nm(ε):
229 (35,800), 284 (22,600), 322(21,000) Infrared absorption spectrum ν max (KBr) cm⁻¹:
3,260, 1,684, 1,626, 1,567, 1,467, 1,288 ¹H-NMR(DMSO-d6)δppm :
2.53(3H,s), 2.55(3H,s), 6.93(1H,s), 6.96(1H,s), 8.17(1H,s),
8.53(1H,s), 9.5-13.0(6H) ¹³C-NMR (DMSO-d₆)δppm:
29.1, 32.1, 102.26, 102.32, 109.9, 112.4, 119.6, 119.8, 120.3, 120.9,
126.3, 132.5, 133.4, 136.2, 141.2, 141.7, 150.4, 150.8, 152.1, 152.68,
152.73, 154.5, 167.4, 167.5, 172.5, 172.9, 199.1, 201.1

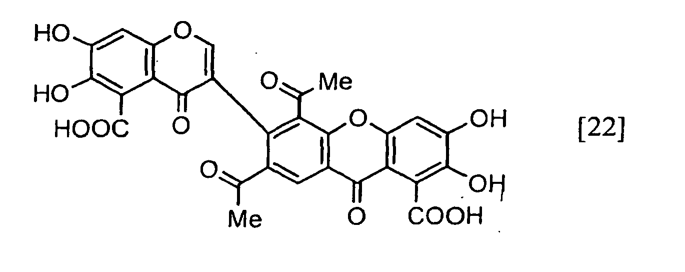

### Example 3

### Preparation Example

The following composition is suspended in a sterilized purified water in 100 ml, and eye drop can be prepared by adjusting pH 7.0 in a concentration isotonic to tears.

| SPF-3059-5 | 50 mg |
|---|---|
| Potassium dihydrogen phosphate | proper quantity |
| Disodium hydrogenphosphate | proper quantity |
| Common salt | proper quantity |
| Benzethonium chloride | 10mg |
| Sterilized purified water | proper quantity |

### Example 4

### Preparation Example

According to a conventional method for eye ointment, eye ointment of the following formulation can be prepared.

| | |
|---|---|
| SPF-3059-5 | 50 mg |
| Liquid paraffin | 10 g |
| White petrolatum | proper quantity |

### Example 5

### Pharmacological Test Example in a rat middle cerebral artery occlusion model

Damage of cerebral nerve cells can be evaluated as the size of infarct region resulted by permanent middle cerebral artery occlusion or middle cerebral artery occlusion - reperfusion. It can be also quantified by the number of nerve fibers and the number of reproduced nerve fibers present in the infarct region or by the number of nerve fibers penetrating into the infarct region from a normal region, which can be used as indicators to know the degree of injury. Pharmacological effect of a therapeutic or preventive agent for ischemic cerebrovascular diseases having suppression effect can be confirmed using these parameters as indicators.

### Test method

### (1) Administration of drug

SPF-3059-5 as a test substance is directly administered to the cerebral infarction region with a mini-osmotic pump (Alza). The test substance is diluted with PBS to 0.1 mg/ml and filled in a reservoir of a pump. PBS is filled in as a control. The pump is incubated in an isotonic sodium chloride solution at 37°C from 2 days before the operation to stabilize the flow rate.

### (2) Operation

Pharmacological efficacy of the test substances is evaluated in a permanent middle cerebral artery occlusion model using a Stroke-Prone Spontaneously Hypertensive Rat (SHRSP rat) and a middle cerebral artery occlusion - reperfusion model using a Wistar rat. It is performed after a cannula implantation operation into the brain cortex parenchyma for administering the test substance.

### Cannula implantation operation

(i) A rat is anesthetized with Halothane and fixed to a brain stereotaxic apparatus.
(ii) Head skin is incised to expose the skull.
(iii) A hole is cut open with a drill in the left skull, and a cannula connected to a mini-osmotic pump which is filled with a drug is implanted in the left brain cortex parenchyma.
(iv) The cannula is fixed to the skull with dental cement and a pump is inserted into the dorsal subcutis. The incised area is sewn up.

### Permanent middle cerebral artery occlusion model

Permanent middle cerebral artery occlusion is caused in a SHRSP rat by the following method.
(i) Muscle at the left side head is removed, temporal bone is drilled to expose middle cerebral artery.
(ii) The middle cerebral artery is cauterized with a bipolar coagulator to block the blood flow.
(iii) The wounded area of the rat is sewn up and revive it while maintaining the body temperature.

### Middle cerebral artery occlusion - reperfusion model

Middle cerebral artery occlusion - reperfusion is caused in a Wistar rat by the following method.
(i) The cervical skin is incised to expose the carotid artery.
(ii) The outside carotid artery is ligated with a silk thread.
(iii) A thin blood vessel connecting the outer carotid artery and inner carotid artery is cauterized to cut off. The total carotid artery is ligated.
(iv) The blood flow of inner carotid artery is temporarily stopped and a hole is opened at the branching site to the outer carotid artery and an embolus (nylon thread coated with silicon) is inserted.
(v) The embolus is inserted further to the depth with a care so as not let it enter the sphenopalatine artery.
(vi) The embolus is ligated and fixed to the blood vessel.
(vii) The wounded area of the rat is sewn up and revive it while maintaining the body temperature.

### (3) Histological evaluation

As a pharmacological evaluation, the size of the infarct region dyed with TTC (abbreviation of 2,3,5-triphenyltetrazolium chloreide) and the number of immunostained nerve fibers and reproduced nerve fibers present in the infarct region are measured.

### Evaluation by TTC staining

(i) The rat is perfused through the heart with a normal saline solution under anesthesia one week after the operation.
(ii) The brain is removed and a section having a thickness of 2 mm was prepared with a brain slicer and attached on a cover glass with a quick setting adhesive.
(iii) The section attached on the cover glass is immersed in 0.8% TCC/PBS solution at 37°C for 10 minutes and dyed.
(iv) It is transferred to a 10% formalin buffer at 4°C and dyeing is terminated.
(v) A stereoscopic microscope photograph is taken, converted to a digital file with a computer and the size of the cerebral infarction is measured by an image analysis software.

### Evaluation by immunostaining

(i) The rat is perfused through the heart with PBS under anesthesia one week after the operation and then perfused and fixed with 4%
   paraformaldehyde/PBS solution.
(ii) The brain is removed and post-fixed with a fixative for post-fix for one night and stored while soaking in 30% sucrose/PBS solution at 4°C.
(iii) A section having a thickness of 40 µm was prepared with a cryostat and collected as a section floating in TBS.
(iv) It is immunostained by ABC method and a slide is prepared. As for antibody, an anti-neurofilament antibody is used as an indicator of nerve fiber and an anti-GAP-43 antibody is for used as an indicator of regenerating nerve fibers.
(v) A picture of infarct region was taken under a microscope, converted to a digital file with a computer and the number of nerve fibers and reproduced nerve fibers and the number of fibers penetrating into the infarct region from a normal region are quantified by an image analysis software.

### INDUSTRIAL APPLICABILITY

The compound of the present invention having a semaphorin inhibitory activity which is obtained by culturing Penicillium sp. SPF-3059 strain shows suppressing effect on nerve cell death involved in ischemic injury and therefore can be advantageously used as a therapeutic or preventive agent for retinal neuropathy associated with ischemic injury such as glaucoma, central retinal artery occlusion, branch retinal artery occlusion, central retinal vein occlusion, branch retinal vein occlusion, ischemic optic neuropathy, diabetic retinopathy, macular degeneration and retinopathy of prematurity, and as a therapeutic or preventive agent for ischemic cerebrovascular disorders such as cerebral embolism, transient cerebral ischemia, subclavian steal syndrome, Wallenberg syndrome (lateral medullary syndrome), cerebral thrombosis, lacunar infarct, reversible ischemic neurological deficit, cerebral infarction, moyamoya disease (occlusion of the circle of Willis), hypoxic encephalopathy, sinus venosus thrombosis or postoperative spinal cord ischemia.

## Claims

1. Use of a compound having a semaphorin inhibitory activity which is obtained by culturing Penicillium sp. SPF-3059 strain and represented by formula [22]: 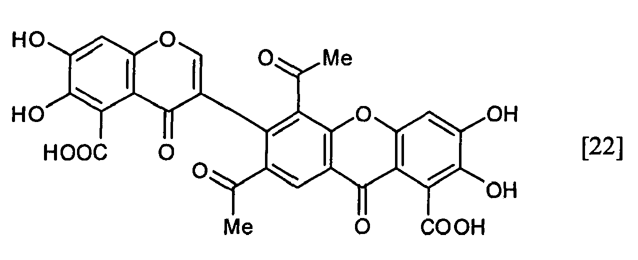 or a pharmaceutically acceptable salt thereof; in the manufacture of a medicament for use as an active ingredient in a therapeutic or preventive agent for suppressing nerve cell death in an ischemic nerve injury by ischemic disorder.

2. The use of a compound or a pharmaceutically acceptable salt thereof in the manufacture of a medicament according to claim 1 wherein the ischemic disorder is cerebral embolism, transient cerebral ischemia, subclavian steal syndrome, Wallenberg syndrome (lateral medullary syndrome), cerebral thrombosis, lacunar infarct, reversible ischemic neurological deficit, cerebral infarction, moyamoya disease (occlusion of the circle of Willis), hypoxic encephalopathy, sinus venosus thrombosis or postoperative spinal cord ischemia.

3. The use of a compound or a pharmaceutically acceptable salt thereof in the manufacture of a medicament according to claim 1 wherein the ischemic disorder is glaucoma, central retinal artery occlusion, branch retinal artery occlusion, central retinal vein occlusion, branch retinal vein occlusion, ischemic optic neuropathy, diabetic retinopathy, macular degeneration or retinopathy of prematurity.

4. The use of a compound or a pharmaceutically acceptable salt thereof in the manufacture of a medicament according to claim 1 wherein the ischemic disorder is diabetic retinopathy.

5. A compound having a semaphorin inhibitory activity which is obtained by culturing Penicillium sp. SPF-3059 strain and represented by formula [22] : 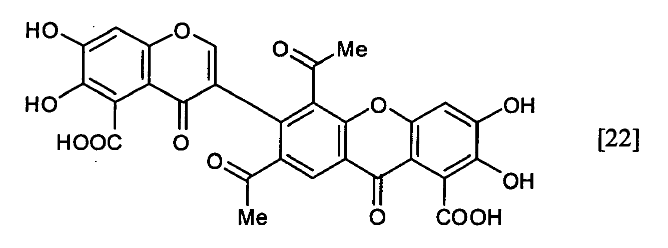 or a pharmaceutically acceptable salt thereof; for use as an active ingredient in a therapeutic or preventive agent for suppressing nerve cell death in an ischemic nerve injury by ischemic disorder.

6. The compound or a pharmaceutically acceptable salt thereof for use as an active ingredient in a therapeutic or preventive agent according to claim 5, wherein the ischemic disorder is cerebral embolism, transient cerebral ischemia, subclavian steal syndrome, Wallenberg syndrome (lateral medullary syndrome), cerebral thrombosis, lacunar infarct, reversible ischemic neurological deficit, cerebral infarction, moyamoya disease (occlusion of the circle of Willis), hypoxic encephalopathy, sinus venosus thrombosis or postoperative spinal cord ischemia.

7. The compound or a pharmaceutically acceptable salt thereof for use as an active ingredient in a therapeutic or preventive agent according to claim 5, wherein the ischemic disorder is glaucoma, central retinal artery occlusion, branch retinal artery occlusion, central retinal vein occlusion, branch retinal vein occlusion, ischemic optic neuropathy, diabetic retinopathy, macular degeneration or retinopathy of prematurity.

8. The compound or a pharmaceutically acceptable salt thereof for use as an active ingredient in a therapeutic or preventive agent according to claim 5, wherein the ischemic disorder is diabetic retinopathy.

## Patentansprüche

1. Verwendung einer Verbindung mit einer Semaphorin-Hemmungsaktivität, die durch Kultivieren des Penicillium sp. SPF-3059-Stammes erhalten wird und durch die Formel [22] dargestellt ist: 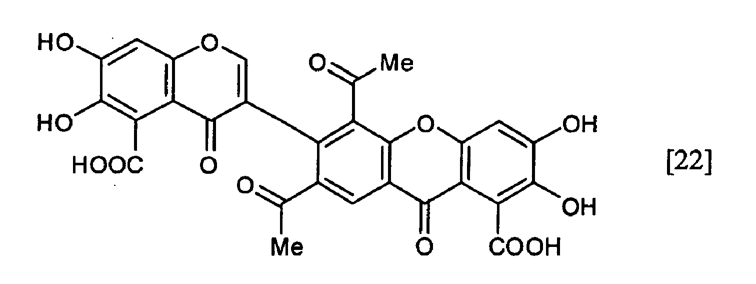 oder eines pharmazeutisch verträglichen Salzes davon; bei der Herstellung eines Medikaments zur Verwendung als ein Wirkstoff in einem Arznei- oder Präventionsmittel zum Unterdrücken des Nervenzellentodes bei einer ischämischen Nervenverletzung durch eine ischämische Störung.

2. Die Verwendung einer Verbindung oder eines pharmazeutisch verträglichen Salzes davon bei der Herstellung eines Medikaments gemäß Anspruch 1, wobei die ischämische Störung zerebrale Embolie, transiente zerebrale Ischämie, Subklavia-Anzapfsyndrom, Wallenberg Syndrom (laterales medulläres Syndrom), zerebrale Thrombose, lakunärer Infarkt, reversibles ischämisches neurologisches Defizit, zerebraler Infarkt, Moyamoya-Erkrankung (Verschluss des Circulus arteriosus), hypoxische Enzephalopathie, Sinus venosus-Thrombose oder postoperative Rückenmarksischämie ist.

3. Die Verwendung einer Verbindung oder eines pharmazeutisch verträglichen Salzes davon bei der Herstellung eines Medikaments gemäß Anspruch 1, wobei die ischämische Störung Glaukom, Verschluss der zentralen Netzhautarterie, Verschluss der Astnetzhautarterie, Verschluss der zentralen Netzhautvene, Verschluss der Astnetzhautvene, ischämische optische Neuropathie, diabetische Retinopathie, Makuladegeneration oder Frühgeborenenretinopathie ist.

4. Die Verwendung einer Verbindung oder eines pharmazeutisch verträglichen Salzes davon bei der Herstellung eines Medikaments gemäß Anspruch 1, wobei die ischämische Störung diabetische Retinopathie ist.

5. Eine Verbindung mit einer Semaphorin-Hemmungsaktivität, die durch Kultivieren des Penicillium sp. SPF-3059-Stammes erhalten wird und durch die Formel [22] dargestellt ist: 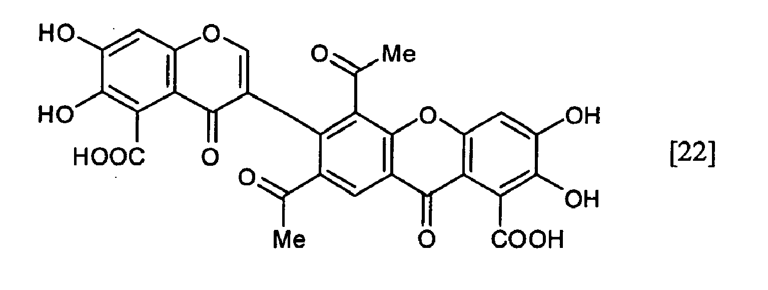 oder ein pharmazeutisch verträgliches Salz davon; zur Verwendung als ein Wirkstoff in einem Arznei- oder Präventionsmittel zum Unterdrücken des Nervenzellentodes bei einer ischämischen Nervenverletzung durch eine ischämische Störung.

6. Die Verbindung oder ein pharmazeutisch verträgliches Salz davon zur Verwendung als ein Wirkstoff in einem Arznei- oder Präventionsmittel gemäß Anspruch 5, wobei die ischämische Störung zerebrale Embolie, transiente zerebrale Ischämie, Subklavia-Anzapfsyndrom, Wallenberg Syndrom (laterales medulläres Syndrom), zerebrale Thrombose, lakunärer Infarkt, reversibles ischämisches neurologisches Defizit, zerebraler Infarkt, Moyamoya-Erkrankung (Verschluss des Circulus arteriosus), hypoxische Enzephalopathie, Sinus venosus-Thrombose oder postoperative Rückenmarksischämie ist.

7. Die Verbindung oder ein pharmazeutisch verträgliches Salz davon zur Verwendung als ein Wirkstoff in einem Arznei- oder Präventionsmittel gemäß Anspruch 5, wobei die ischämische Störung Glaukom, Verschluss der zentralen Netzhautarterie, Verschluss der Astnetzhautarterie, Verschluss der zentralen Netzhautvene, Verschluss der Astnetzhautvene, ischämische optische Neuropathie, diabetische Retinopathie, Makuladegeneration oder Frühgeborenenretinopathie ist.

8. Die Verbindung oder ein pharmazeutisch verträgliches Salz davon zur Verwendung als ein Wirkstoff in einem Arznei- oder Präventionsmittel gemäß Anspruch 5, wobei die ischämische Störung diabetische Retinopathie ist.

## Revendications

1. Utilisation d'un composé présentant une activité inhibitrice de la sémaphorine qui est obtenu en réalisant la culture de la souche SPF-3059 de l'espèce *Pénicillium* et qui est représenté par la formule [22] : 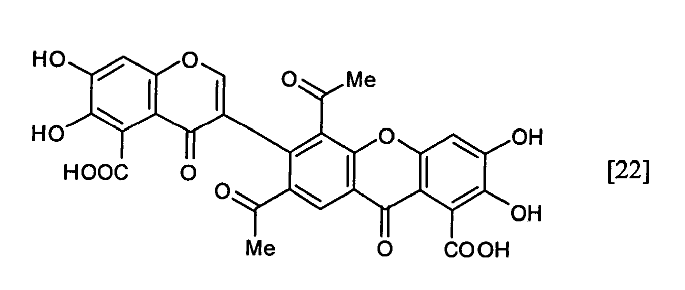 ou un sel pharmaceutiquement acceptable de celui-ci dans la fabrication d'un médicament destiné à être utilisé comme principe actif dans un agent thérapeutique ou de prévention dont le but est de supprimer la mort des cellules nerveuses dans une lésion nerveuse ischémique provoquée par un trouble ischémique.

2. Utilisation d'un composé ou d'un sel pharmaceutiquement acceptable de celui-ci dans la fabrication d'un médicament selon la revendication 1, dans laquelle le trouble ischémique est l'embolie cérébrale, l'accident ischémique cérébral transitoire, le syndrome de vol de la sous-clavière, le syndrome de Wallenberg (syndrome de l'artère latérale du bulbe), la thrombose cérébrale, l'infarctus lacunaire, le déficit neurologique ischémique réversible, l'infarctus cérébral, la maladie de Moya-Moya (occlusion du cercle de Willis), l'encéphalopathie hypoxique, la thrombose des sinus veineux ou l'ischémie post-opératoire de la moelle épinière.

3. Utilisation d'un composé ou d'un sel pharmaceutiquement acceptable de celui-ci dans la fabrication d'un médicament selon la revendication 1, dans laquelle le trouble ischémique est le glaucome, l'occlusion de l'artère centrale de la rétine, l'occlusion d'une branche de l'artère centrale de la rétine, l'occlusion de la veine centrale de la rétine, l'occlusion d'une branche de la veine centrale de la rétine, la neuropathie optique ischémique, la rétinopathie diabétique, la dégénérescence maculaire ou la rétinopathie du prématuré.

4. Utilisation d'un composé ou d'un sel pharmaceutiquement acceptable de celui-ci dans la fabrication d'un médicament selon la revendication 1, dans laquelle le trouble ischémique est la rétinopathie diabétique.

5. Composé présentant une activité inhibitrice de la sémaphorine qui est obtenu en réalisant la culture de la souche SPF-3059 de l'espèce *Pénicillium* et qui est représenté par la formule [22] : 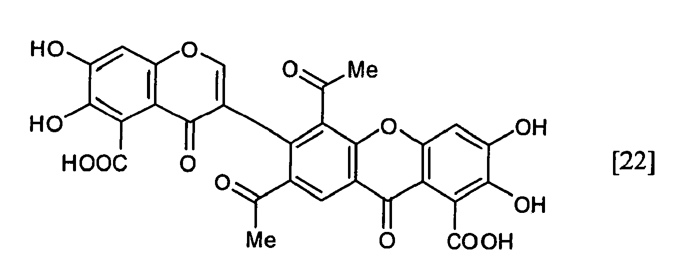 ou sel pharmaceutiquement acceptable de celui-ci destiné à être utilisé comme principe actif dans un agent thérapeutique ou de prévention dont le but est de supprimer la mort des cellules nerveuses dans une lésion nerveuse ischémique provoquée par un trouble ischémique.

6. Composé ou sel pharmaceutiquement acceptable de celui-ci destiné à être utilisé comme principe actif dans un agent thérapeutique ou de prévention selon la revendication 5, dans lequel le trouble ischémique est l'embolie cérébrale, l'accident ischémique cérébral transitoire, le syndrome de vol de la sous-clavière, le syndrome de Wallenberg (syndrome de l'artère latérale du bulbe), la thrombose cérébrale, l'infarctus lacunaire, le déficit neurologique ischémique réversible, l'infarctus cérébral, la maladie de Moya-Moya (occlusion du cercle de Willis), l'encéphalopathie hypoxique, la thrombose des sinus veineux ou l'ischémie post-opératoire de la moelle épinière.

7. Composé ou sel pharmaceutiquement acceptable de celui-ci destiné à être utilisé comme principe actif dans un agent thérapeutique ou de prévention selon la revendication 5, dans lequel le trouble ischémique est le glaucome, l'occlusion de l'artère centrale de la rétine, l'occlusion d'une branche de l'artère centrale de la rétine, l'occlusion de la veine centrale de la rétine, l'occlusion d'une branche de la veine centrale de la rétine, la neuropathie optique ischémique, la rétinopathie diabétique, la dégénérescence maculaire ou la rétinopathie du prématuré.

8. Composé ou sel pharmaceutiquement acceptable de celui-ci destiné à être utilisé comme principe actif dans un agent thérapeutique ou de prévention selon la revendication 5, dans laquelle le trouble ischémique est la rétinopathie diabétique.
